(19) Europäisches Patentamt
European Patent Office
Office européen des brevets

(11) Veröffentlichungsnummer: **0 159 576**
**A2**

(12) # EUROPÄISCHE PATENTANMELDUNG

(21) Anmeldenummer: **85103839.8**

(22) Anmeldetag: **29.03.85**

(51) Int. Cl.⁴: **C 07 D 251/22**, C 07 D 251/16, C 07 D 251/46, C 07 D 239/42, C 07 D 239/47, C 07 D 239/52, C 07 D 249/14, A 01 N 47/36

(30) Priorität: **10.04.84 DE 3413490**

(43) Veröffentlichungstag der Anmeldung: **30.10.85 Patentblatt 85/44**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Stetter, Jörg, Dr., Gellertweg 4, D-5600 Wuppertal 1 (DE)**
Erfinder: **Schmidt, Robert R. Dr., Im Waldwinkel 110, D-5060 Bergisch-Galdbach 2 (DE)**
Erfinder: **Santel, Hans-Joachim, Dr., Gerstenkamp 19, D-5000 Köln 80 (DE)**
Erfinder: **Hänssler, Gerd, Dr., Heymannstrasse 40, D-5090 Leverkusen 1 (DE)**
Erfinder: **Eue, Ludwig, Dr., Paul-Klee-Strasse 36, D-5090 Leverkusen 1 (DE)**

(54) **2-(Alkoximinoalkyloxycarbonyl)-phenylsulfonylharnstoffe.**

(57) Die Erfindung betrifft neue 2-(Alkoximinoalkyloxycarbonyl)-phenylsulfonylharnstoffe der Formel (I)

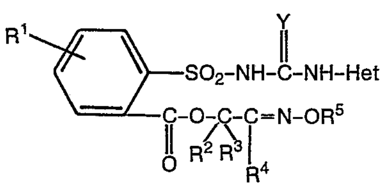

(I)

worin
Y für Sauerstoff oder Schwefel steht und
Het für einen heterocyclischen Rest der Formel

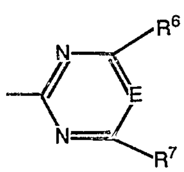

oder

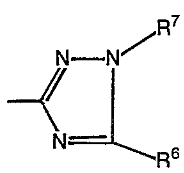

steht, wobei
E für Stickstoff oder die CH-Gruppe steht und die Reste $R^1$–$R^7$ die in der Beschreibung genannte Bedeutung haben, Verfahren zu ihrer Herstellung und ihre Verwendung als Herbizide und Fungizide.

BAYER AKTIENGESELLSCHAFT 5090 Leverkusen, Bayerwerk
Konzernverwaltung RP
Patentabteilung Bi/AB
Ib

2-(Alkoximinoalkyloxycarbonyl)-phenylsulfonylharnstoffe

Die Erfindung betrifft neue 2-(Alkoximinoalkyloxycarbonyl)-phenylsulfonylharnstoffe, mehrere Verfahren zu ihrer Herstellung und ihre Verwendung als Herbizide und Fungizide.

Es ist bereits bekannt, daß bestimmte 2-(Oxycarbonyl)-phenylsulfonylharnstoffe, wie beispielsweise der N-[2-(Methoxycarbonyl)-phenylsulfonyl]-N'-(4,6-dimethylpyrimidin-2-yl)harnstoff, herbizide Eigenschaften besitzen. (vergl. US 4 394 506 oder EP-OS 34 431).

Die herbizide Wirkung dieser Verbindungen gegenüber wichtigen Schadpflanzen, ebenso wie deren Verträglichkeit gegenüber wichtigen Kulturpflanzen ist jedoch nicht immer in allen Anwendungsbereichen völlig zufriedenstellend.

Ueber eine fungizide Wirkung der vorbekannten 2-(Oxycarbonyl) phenylsulfonylharnstoffe ist noch nichts bekannt.

Es wurden neue 2-(Alkoximinoalkyloxycarbonyl)-phenylsulfonylharnstoffe der allgemeinen Formel (I)

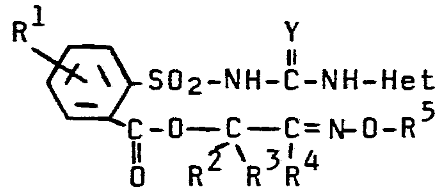

(I)

in welcher

$R^1$ für Wasserstoff, Halogen, Alkyl, Halogenalkyl, Alkoxy oder Nitro steht,

$R^2$ für Wasserstoff, Alkyl oder gegebenenfalls substituiertes Aryl steht,

$R^3$ für Wasserstoff oder Alkyl steht,

$R^4$ für Wasserstoff, Alkyl, Alkoxy, Alkylthio, Alkoxyalkyl, Alkylthioalkyl, Cyano, Alkoxycarbonyl oder gegebenenfalls substituiertes Aryl steht,

$R^5$ für Wasserstoff, Alkyl, Alkenyl, Alkinyl, Halogenalkyl, Halogenalkenyl, Alkoxyalkyl, Alkylthioalkyl oder für gegebenenfalls substituiertes Aralkyl steht,

Y für Sauerstoff oder Schwefel steht und

Het für einen heterocyclischen Rest der Formel

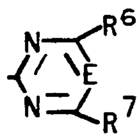 oder 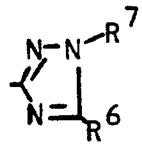 steht,

wobei

E für Stickstoff oder die CH-Gruppe steht und

$R^6$ und $R^7$ unabhängig voneinander jeweils für Alkyl, Alkoxy oder Alkoxyalkyl stehen,

gefunden.

Weiterhin wurde gefunden, daß man die neuen 2-(Alkoximinoalkyloxycarbonyl)-phenylsulfonylharnstoffe der allgemeinen Formel (I),

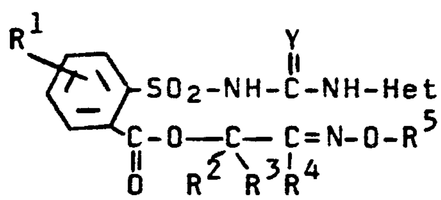 (I)

in welcher

$R^1$ für Wasserstoff, Halogen, Alkyl, Halogenalkyl, Alkoxy oder Nitro steht,

$R^2$ für Wasserstoff, Alkyl oder gegebenenfalls substituiertes Aryl steht,

$R^3$ für Wasserstoff oder Alkyl steht,

$R^4$ für Wasserstoff, Alkyl, Alkoxy, Alkylthio, Alkoxyalkyl, Alkylthioalkyl, Cyano, Alkoxycarbonyl oder gegebenenfalls substituiertes Aryl steht,

$R^5$ für Wasserstoff, Alkyl, Alkenyl, Alkinyl, Halogenalkyl, Halogenalkenyl, Alkoxyalkyl, Alkylthioalkyl oder für gegebenenfalls substituiertes Aralkyl steht,

Y für Sauerstoff oder Schwefel steht und

Het für einen heterocyclischen Rest der Formel

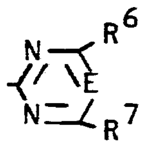 oder 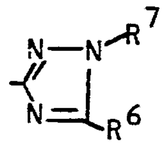 steht,

wobei

E für Stickstoff oder die CH-Gruppe steht und

$R^6$ und $R^7$ unabhängig voneinanander jeweils für Alkyl, Alkoxy oder Alkoxyalkyl stehen,

erhält, wenn man

a) heterocyclische Aminoverbindungen der Formel (II),

$$Het\text{-}NH_2 \qquad (II)$$

in welcher

Het die oben angegebene Bedeutung hat,

mit 2-(Alkoximinoalkyloxycarbonyl)-phenylsulfonyl-iso-(thio)cyanaten der Formel (III),

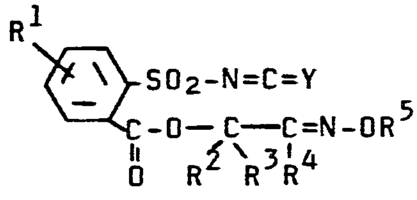

(III)

in welcher

$R^1$, $R^2$, $R^3$, $R^4$, $R^5$ und Y die oben angegebene Bedeutung haben,

gegebenenfalls in Gegenwart eines Verdünnungsmittels und gegebenenfalls in Gegenwart eines basischen Katalysators umsetzt, oder wenn man

b) 2-(Alkoximinoalkyloxycarbonyl)-phenylsulfonamide der Formel (IV),

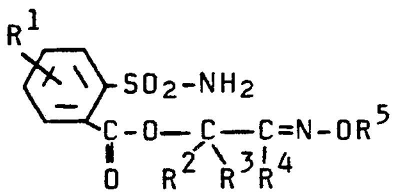

(IV)

in welcher

$R^1$, $R^2$, $R^3$, $R^4$ und $R^5$ die oben angegebene Bedeutung haben,

mit (Thio)Carbamaten der Formel (V),

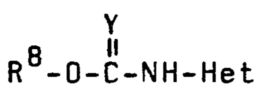

(V)

in welcher

Y und Het die oben angegebene Bedeutung haben und

$R^8$ für Alkyl oder Aryl steht,

jeweils gegebenenfalls in Gegenwart eines Verdünnungsmittels und jeweils gegebenenfalls in Gegenwart einer Base umsetzt.

Schließlich wurde gefunden, daß die neuen 2-(Alkoximinoalkyloxycarbonyl)-phenylsulfonylharnstoffe der Formel (I) herbizide, insbesondere auch selektiv-herbizide und fungizide Eigenschaften besitzen.

Ueberraschenderweise besitzen die neuen 2-(Alkoximinoalkyloxycarbonyl)-phenylsulfonylharnstoffe der Formel (I) bei gleich guter herbizider Wirkung gegen wichtige Schadpflanzen eine erheblich bessere Verträglichkeit gegenüber wichtigen Kulturpflanzen im Vergleich zu den aus dem Stand der Technik bekannten 2-(Oxycarbonyl)phenylsulfonylharnstoffen, wie beispielsweise dem N-[2-(Methoxycarbonyl)-phenylsulfonyl]-N'-(4,6-dimethylpyrimidin-2-yl)-harnstoff, welcher eine chemisch und wirkungsmäßig naheliegende Verbindung ist.

Darüberhinaus besitzen die neuen 2-(Alkoximinoalkyloxycarbonyl)-phenylsulfonylharnstoffe der Formel (I) zusätzlich völlig überraschenderweise eine hervorragende fungizide Wirkung.

Die neuen 2-(Alkoximinoalkyloxycarbonyl)-phenylsulfonylharnstoffe sind durch die Formel (I) allgemein definiert. Bevorzugt sind die Verbindungen der Formel (I), bei welchen

$R^1$ für Wasserstoff, Fluor, Chlor, Brom, Jod, Trifluormethyl, Nitro, sowie jeweils geradkettiges oder verzweigtes Alkyl oder Alkoxy mit jeweils bis zu 4 Kohlenstoffatomen steht,

$R^2$ für Wasserstoff, für geradkettiges oder verzweigtes Alkyl mit bis zu 8 Kohlenstoffatomen oder für einfach oder mehrfach, gleich oder verschieden substituiertes Aryl

mit 6 bis 10 Kohlenstoffatomen steht, wobei als Arylsubstituenten infrage kommen:
Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen,

$R^3$ für Wasserstoff oder für geradkettiges oder verzweigtes Alkyl mit bis zu 4 Kohlenstoffatomen steht,

$R^4$ für Wasserstoff, Cyano oder für jeweils geradkettiges oder verzweigtes Alkyl, Alkoxy, Alkylthio, Alkoxyalkyl, Alkylthioalkyl oder Alkoxycarbonyl mit jeweils bis zu 6 Kohlenstoffatomen in den einzelnen Alkylteilen oder für gegebenenfalls einfach oder mehrfach gleich oder verschieden substituiertes Aryl mit 6 bis 10 Kohlenstoffatomen steht, wobei als Arylsubstituenten die bei $R^2$ genannten infrage kommen,

$R^5$ für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 6 Kohlenstoffatomen in den einzelnen Alkyl- bzw. Alkenyl- oder Alkinylteilen und gegebenenfalls bis zu 9 gleichen oder verschiedenen Halogenatomen steht oder für geradkettiges oder verzweigtes, gegebenenfalls im Arylteil einfach oder mehrfach, gleich oder verschieden substituiertes Aralkyl mit bis zu 4 Kohlenstoffatomen im Alkylteil und 6 bis 10 Kohlenstoffatomen im Arylteil steht, wobei als Arylsubstituenten die bei $R^2$ genannten infrage kommen,

Y für Sauerstoff oder Schwefel steht und

Het für einen heterocyclischen Rest der Formel

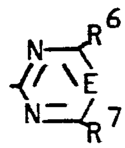 oder 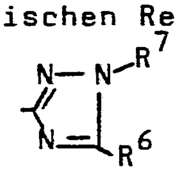 steht,

wobei E für Stickstoff oder die CH-Gruppe steht und $R^6$ und $R^7$ unabhängig voneinander für jeweils geradkettiges oder verzweigtes Alkyl, Alkoxy oder Alkoxyalkyl mit jeweils bis zu 6 Kohlenstoffatomen in den einzelnen Alkylteilen stehen.

Besonders bevorzugt sind Verbindungen der Formel (I), bei welchen

$R^1$ für Wasserstoff, Fluor, Chlor, Brom, Nitro, Methyl, Ethyl, n- oder i-Propyl, Trifluormethyl, Methoxy oder Ethoxy steht,

$R^2$ für Wasserstoff, Methyl, Ethyl, n- oder i-Propyl, n-, i-, s- oder t-Butyl, sowie für gegebenenfalls ein- bis dreifach gleich oder verschieden durch Fluor, Chlor, Brom, Cyano, Methyl, Ethyl, Methoxy, Ethoxy oder Trifluormethyl substituiertes Phenyl steht,

$R^3$ für Wasserstoff, Methyl, Ethyl, n- oder i-Propyl, n-, i-, s- oder t-Butyl steht,

$R^4$ für Wasserstoff, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methoxymethyl, Methoxyethyl, Ethoxyethyl, Methylthiomethyl, Methylthioethyl, Ethylthioethyl, Methoxycarbonyl, Ethoxycarbonyl sowie für gegebenenfalls ein- bis dreifach gleich oder verschieden durch Fluor, Chlor, Brom, Cyano, Nitro, Methyl, Ethyl, Methoxy, Ethoxy oder Trifluormethyl substituiertes Phenyl steht,

$R^5$ für Wasserstoff, Methyl, Ethyl, n- oder i-Propyl, n-, i-, s- oder t-Butyl, Allyl, Butenyl, Propargyl, Methoxymethyl, Methoxyethyl, Ethoxyethyl, Methylthiomethyl, Methylthioethyl, Ethylthioethyl, Chlorallyl, Dichlorallyl oder für jeweils gegebenenfalls ein- bis dreifach, gleich oder verschieden im Phenylteil durch Fluor, Chlor, Brom, Cyano, Nitro, Methyl, Ethyl, Methoxy, Ethoxy oder Trifluormethyl substituiertes Benzyl oder Phenylethyl steht,

Y für Sauerstoff oder Schwefel steht und

Het für einen heterocyclischen Rest der Formel

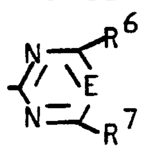 oder 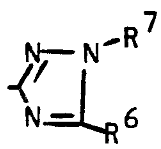 steht,

wobei E für Stickstoff oder die CH-Gruppe steht und $R^6$ und $R^7$ unabhängig voneinander jeweils für Methyl, Ethyl, Methoxy, Ethoxy, Methoxymethyl, Ethoxymethyl, Methoxyethyl oder Ethoxyethyl stehen.

Im einzelnen seien außer den bei den Herstellungsbeispielen genannten Verbindungen die folgenden Sulfonylharnstoffe der allgemeinen Formel (I) genannt:

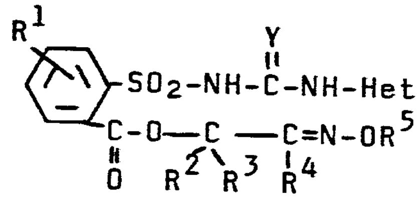

(I)

Tabelle 1

| $R^1$ | $R^2$ | $R^3$ | $R^4$ | $R^5$ | Y | Het |
|---|---|---|---|---|---|---|
| H | $CH_3$ | H | H | $CH_3$ | S | 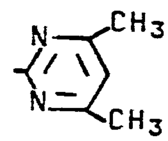 |
| H | $CH_3$ | H | H | H | S | 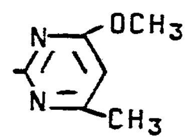 |
| H | $CH_3$ | H | H | $CH_3$ | O | 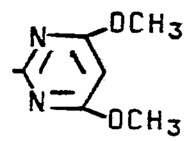 |
| H | $CH_3$ | H | H | $CH_3$ | O | 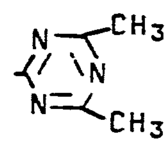 |
| H | $CH_3$ | H | H | $CH_3$ | S | 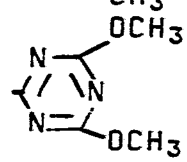 |
| H | $CH_3$ | H | H | $CH_3$ | O | 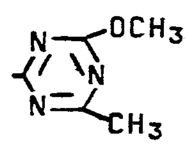 |

Tabelle 1 (Fortsetzung)

| $R^1$ | $R^2$ | $R^3$ | $R^4$ | $R^5$ | Y | Het |
|---|---|---|---|---|---|---|
| H | $CH_3$ | H | H | $CH_3$ | S | 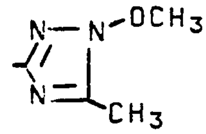 |
| H | $CH_3$ | $CH_3$ | H | $CH_3$ | O | 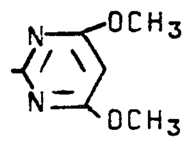 |
| H | $CH_3$ | $CH_3$ | H | $CH_3$ | O | 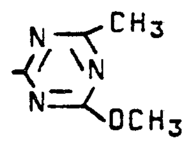 |
| H | $CH_3$ | $CH_3$ | H | $CH_3$ | O | 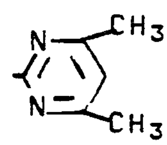 |
| H | $CH_3$ | $CH_3$ | H | $CH_3$ | O | 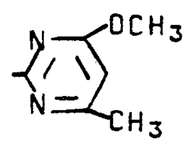 |
| H | $CH_3$ | $CH_3$ | H | $CH_3$ | S | 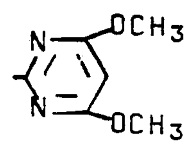 |
| H | $CH_3$ | $CH_3$ | H | $CH_3$ | O | 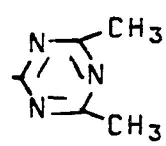 |
| H | $CH_3$ | $CH_3$ | H | $CH_3$ | S | 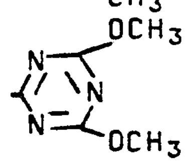 |
| H | $CH_3$ | $CH_3$ | H | $CH_3$ | O | 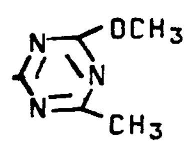 |

Tabelle 1 (Fortsetzung)

| $R^1$ | $R^2$ | $R^3$ | $R^4$ | $R^5$ | Y | Het |
|---|---|---|---|---|---|---|
| H | $CH_3$ | $CH_3$ | H | $CH_3$ | O | 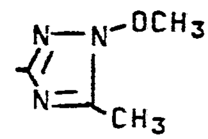 |
| H | $CH_3$ | H | $CH_3$ | $CH_3$ | O | 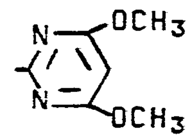 |
| H | $CH_3$ | H | $CH_3$ | $CH_3$ | O | 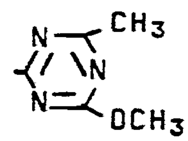 |
| H | H | H | $CH_3$ | $CH_3$ | S | 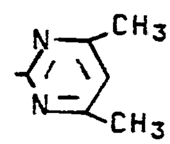 |
| H | H | H | $CH_3$ | $CH_3$ | S | 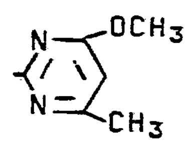 |
| H | H | H | $CH_3$ | $CH_3$ | O | 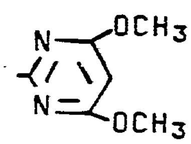 |
| H | H | H | $CH_3$ | $CH_3$ | O | 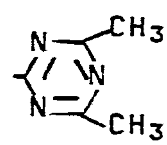 |
| H | H | H | $CH_3$ | $CH_3$ | O | 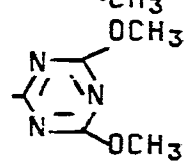 |
| H | H | H | $CH_3$ | $CH_3$ | O | 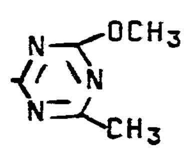 |
| H | H | H | $CH_3$ | $CH_3$ | S | 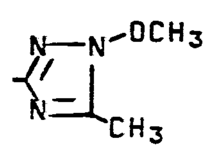 |

Tabelle 1 (Fortsetzung)

| $R^1$ | $R^2$ | $R^3$ | $R^4$ | $R^5$ | Y | Het |
|---|---|---|---|---|---|---|
| H | H | H | $CH_3$ | $CH_3$ | S | 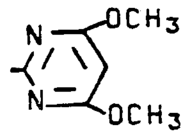 |
| H | H | H | $CH_3$ | $CH_3$ | S | 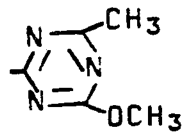 |
| H | H | H | H | $CH_3$ | S | 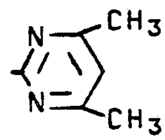 |
| H | H | H | H | $CH_3$ | O | 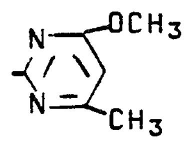 |
| H | H | H | H | $CH_3$ | O | 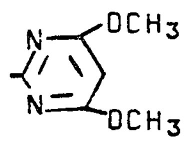 |
| H | H | H | H | $CH_3$ | O | 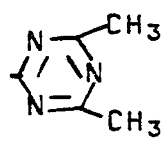 |
| H | H | H | H | $CH_3$ | O | 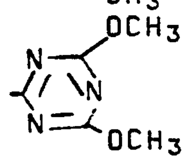 |
| H | H | H | H | $CH_3$ | O | 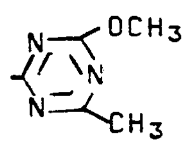 |
| H | H | H | H | $CH_3$ | O | 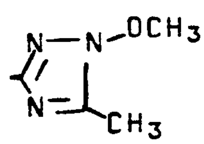 |
| H | H | H | H | $CH_3$ | S | 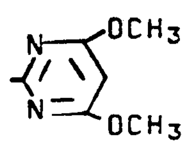 |

Tabelle 1 (Fortsetzung)

| $R^1$ | $R^2$ | $R^3$ | $R^4$ | $R^5$ | Y | Het |
|---|---|---|---|---|---|---|
| H | H | H | H | $CH_3$ | S | 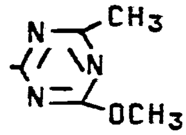 |
| H | $CH_3$ | $CH_3$ | $CH_3$ | $CH_3$ | O | 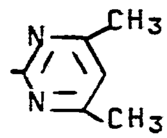 |
| H | $CH_3$ | $CH_3$ | $CH_3$ | $CH_3$ | O | 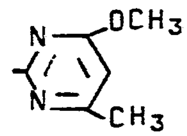 |
| H | $CH_3$ | $CH_3$ | $CH_3$ | $CH_3$ | O | 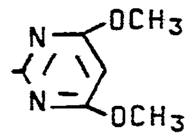 |
| H | $CH_3$ | $CH_3$ | $CH_3$ | $CH_3$ | O | 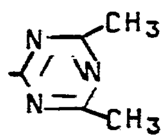 |
| H | $CH_3$ | $CH_3$ | $CH_3$ | $CH_3$ | O | 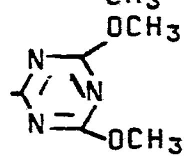 |
| H | $CH_3$ | $CH_3$ | $CH_3$ | $CH_3$ | O | 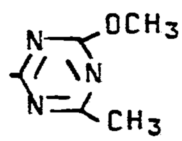 |
| H | $CH_3$ | $CH_3$ | $CH_3$ | $CH_3$ | O | 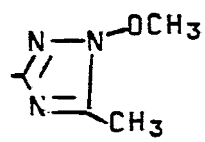 |
| H | $CH_3$ | $CH_3$ | $CH_3$ | $CH_3$ | S | 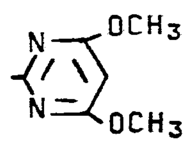 |
| H | $CH_3$ | $CH_3$ | $CH_3$ | $CH_3$ | S | 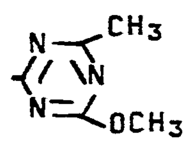 |

Tabelle 1 (Fortsetzung)

| $R^1$ | $R^2$ | $R^3$ | $R^4$ | $R^5$ | Y | Het |
|---|---|---|---|---|---|---|
| H | H | H | H | $C_2H_5$ | O | 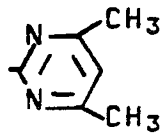 |
| H | H | H | H | $C_2H_5$ | O | 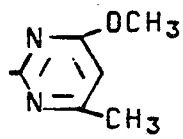 |
| H | H | H | H | $C_2H_5$ | O | 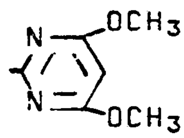 |
| H | H | H | H | $C_2H_5$ | O | 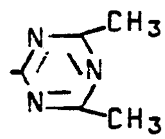 |
| H | H | H | H | $C_2H_5$ | O | 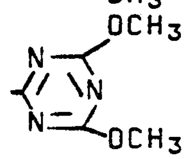 |
| H | H | H | H | $C_2H_5$ | O | 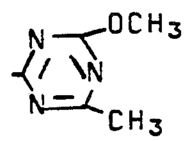 |
| H | H | H | H | $C_2H_5$ | O | 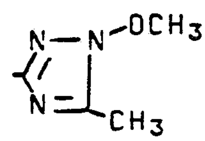 |
| H | H | H | H | $C_2H_5$ | S | 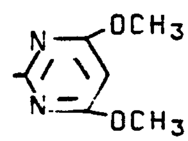 |
| H | H | H | H | $C_2H_5$ | S | 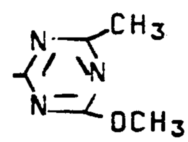 |

Tabelle 1 (Fortsetzung)

| $R^1$ | $R^2$ | $R^3$ | $R^4$ | $R^5$ | Y | Het |
|---|---|---|---|---|---|---|
| H | H | H | H | $-CH_2-CH=CH_2$ | O | 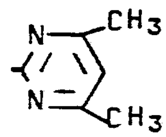 |
| H | H | H | H | $-CH_2-CH=CH_2$ | O | 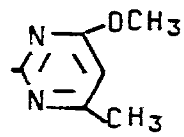 |
| H | H | H | H | $-CH_2-CH=CH_2$ | O | 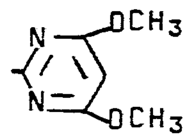 |
| H | H | H | H | $-CH_2-CH=CH_2$ | O | 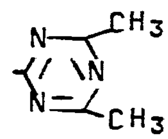 |
| H | H | H | H | $-CH_2-CH=CH_2$ | O | 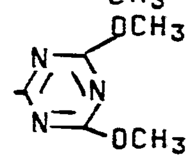 |
| H | H | H | H | $-CH_2-CH=CH2$ | O | 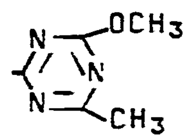 |
| H | H | H | H | $-CH_2-CH=CH_2$ | O | 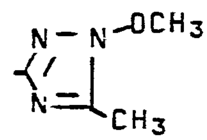 |
| H | H | H | H | $n-C_3H_7$ | O | 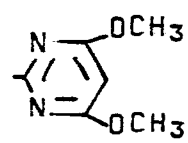 |
| H | H | H | H | $n-C_3H_7$ | O | 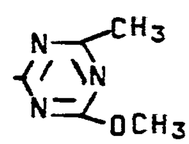 |

Tabelle 1 (Fortsetzung)

| $R^1$ | $R^2$ | $R^3$ | $R^4$ | $R^5$ | Y | Het |
|---|---|---|---|---|---|---|
| H | $CH_3$ | H | H | $-CH_2-CH=CH_2$ | O | 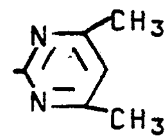 |
| H | $CH_3$ | H | H | $-CH_2-CH=CH_2$ | O | 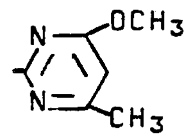 |
| H | $CH_3$ | H | H | $-CH_2-CH=CH_2$ | O | 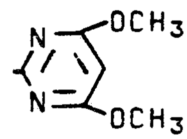 |
| H | $CH_3$ | H | H | $-CH_2-CH=CH_2$ | O | 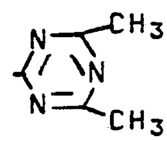 |
| H | $CH_3$ | H | H | $-CH_2-CH=CH_2$ | O | 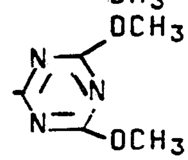 |
| H | $CH_3$ | H | H | $-CH_2-CH=CH_2$ | O | 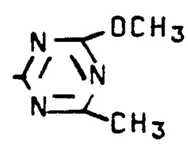 |
| H | $CH_3$ | H | H | $-CH_2-CH=CH_2$ | O | 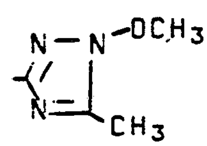 |
| H | $CH_3$ | H | H | $n-C_3H_7$ | O | 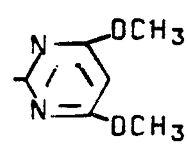 |
| H | $CH_3$ | H | H | $n-C_3H_7$ | O | 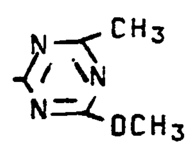 |

Verwendet man beispielsweise 2-[(2-Methoximinoethyl)-oxycarbonyl]-phenylsulfonylisocyanat und 2-Amino-4,6-dimethoxytriazin als Ausgangsstoffe, so läßt sich der Reaktionsablauf des erfindungsgemäßen Verfahrens

a) durch das folgende Formelschema darstellen:

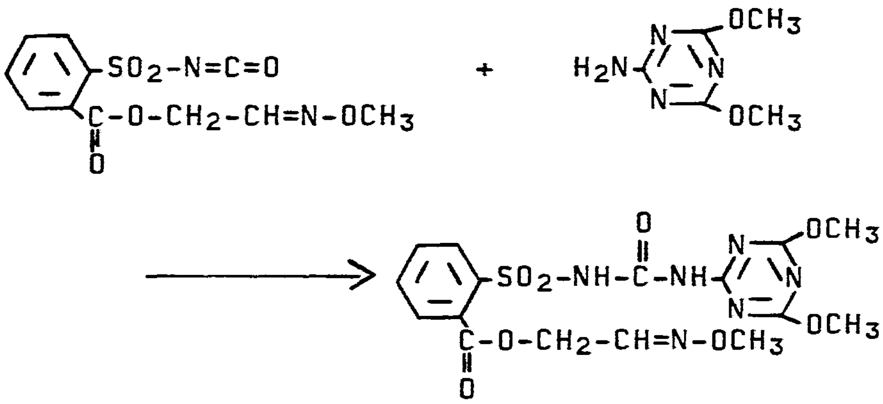

Verwendet man beispielsweise 2-[(2-Methoximinoethyl)-oxycarbonyl]-phenylsulfonsäureamid und N-(4-Methoxy-6-methylpyrimidin-2-yl)-O-phenyl-carbamat als Ausgangsstoffe, so läßt sich der Reaktionsablauf des erfindungsgemäßen Verfahrens

b) durch das folgende Formelschema darstellen:

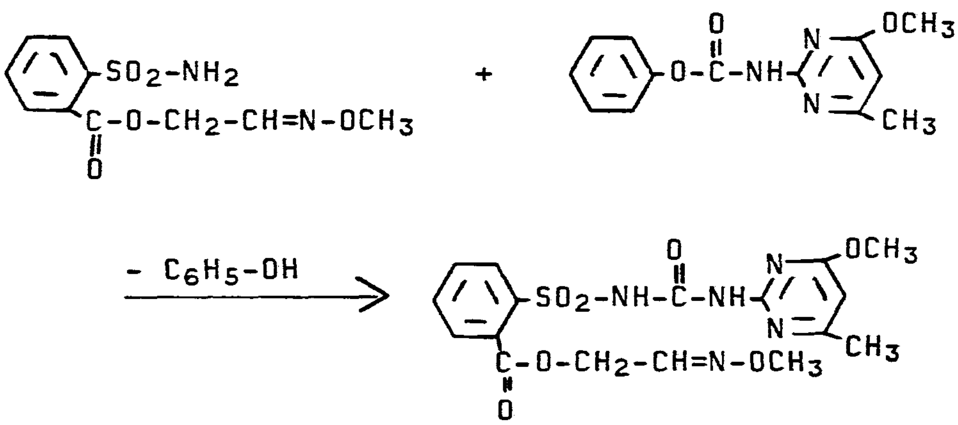

Die als Ausgangsstoffe zur Durchführung des erfindungsgemäßen Verfahrens (a) benötigten heterocyclischen Aminoverbindungen sind durch die Formel (II) allgemein definiert. In dieser Formel (II) steht Het vorzugsweise für diejenigen Reste, die schon bei der Beschreibung der erfindungsgemäßen Stoffe der Formel (I) als bevorzugt für diesen Substituenten genannt wurden.

Die heterocyclischen Aminoverbindungen der Formel (II) sind bekannt (vergl. z. B. EP 73 627, EP 73 562, EP 85 028, US-PS 4 127 405).

Die weiterhin zur Durchführung des erfindungsgemäßen Verfahrens (a) als Ausgangsstoffe benötigten 2-(Alkoximinoalkyloxycarbonyl)-phenylsulfonyliso(thio)cyanate sind durch die Formel (III) allgemein definiert. In dieser Formel (III) stehen $R^1$, $R^2$, $R^3$, $R^4$, $R^5$ und Y vorzugsweise für diejenigen Reste, die schon bei der Beschreibung der erfindungsgemäßen Stoffe der Formel (I) als bevorzugt für diese Substituenten genannt wurden.

Die 2-(Alkoximinoalkyloxycarbonyl)phenylsulfonyliso(thio)-cyanate der Formel (III) sind noch nicht bekannt. Man erhält sie, wenn man 2-(Alkoximinoalkyloxycarbonyl)-phenylsulfonamide der Formel (IV),

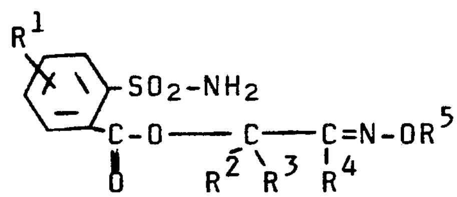

(IV)

in welcher

$R^1$, $R^2$, $R^3$, $R^4$ und $R^5$ die oben angegebene Bedeutung haben,

mit Phosgen oder Thiophosgen oder mit Chlorsufonylisocyanat der Formel (VI),

$$Cl-SO_2-N=C=O \qquad (VI)$$

gegebenenfalls in Gegenwart eines Verdünnungsmittels wie beispielsweise Chloroform oder Chlorbenzol und gegebenenfalls in Gegenwart eines Säurebindemittels wie beispielsweise Triethylamin bei Temperaturen zwischen -30 °C und +150 °C umsetzt.

Die zur Durchführung des erfindungsgemäßen Verfahrens (b) und zur Herstellung der Ausgangsstoffe der Formel (III) als Vorprodukte benötigten 2-(Alkoximinoalkyloxycarbonyl)-phenylsulfonamide sind durch die Formel (IV) allgemein definiert. In dieser Formel (IV) stehen $R^1$, $R^2$, $R^3$, $R^4$ und $R^5$ vorzugsweise für diejenigen Reste, die schon bei der Beschreibung der erfindungsgemäßen Stoffe der Formel (I) als bevorzugt für diese Substituenten genannt wurden.

Die 2-(Alkoximinoalkyloxycarbonyl)-phenylsulfonamide der Formel (IV) sind ebenfalls noch nicht bekannt. Man erhält sie, wenn man 2-Carboxyphenylsulfonamide der Formel (VII),

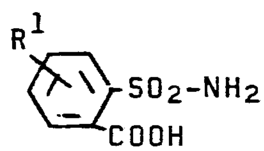

(VII)

in welcher

$R^1$ die oben angegebene Bedeutung hat,

mit Oximinoalkylverbindungen der Formel (VIII),

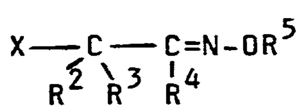

(VIII)

in welcher

$R^2$, $R^3$, $R^4$ und $R^5$ die oben angegebene Bedeutung haben und

X für eine elektonenanziehende Abgangsgruppe, wie beispielsweise Halogen, insbesondere Chlor oder Brom, oder jeweils gegebenenfalls substituiertes Arylsulfonyloxy, Alkylsulfonyloxy oder Alkoxysulfonyloxy, wie insbesondere p-Toluolsulfonyloxy, Methansulfonyloxy oder Methoxysulfonyloxy steht,

gegebenenfalls in Gegenwart eines Verdünnungsmittels wie beispielsweise Acetonitril und gegebenenfalls in Gegenwart eines Säurebindemittels wie beispielsweise Triethylamin bei Temperaturen zwischen 0 °C und +120 °C umsetzt.

Die zur Durchführung des erfindungsgemäßen Verfahrens (b) weiterhin als Ausgangsstoffe benötigten (Thio)Carbamate sind durch die Formel (V) allgemein definiert. In dieser Formel (V) stehen Y und Het vorzugsweise für diejenigen Reste, die schon bei der Beschreibung der erfindungsgemäßen Stoffe der Formel (I) vorzugsweise für diese Substituenten genannt wurden. $R^8$ steht vorzugsweise für Methyl, Ethyl oder Phenyl.
Die (Thio)Carbamate der Formel (V) sind ebenfalls bekannt, (vergl. z. B. EP 70 802, EP 70 804, EP 71 958, EP 72 347 oder EP 79 683) oder lassen sich nach bekannten Methoden nach einfachen Analogieverfahren herstellen.

Die Oximinoalkylverbindungen der Formel (VIII) sind ebenfalls bekannt, (vergl. z. B. DE-OS 2 922 759 oder K.A. Ogloblin, V.P. Semenov, Zhurnal Organicheskoi Khimii, Vol. 1, Nr. 8

1361 - 1364, oder K.A. Obloblin, A.A. Potekhin, Zhurnal Obshchei Khimii, Vol. 34, Nr. 8, 2688 - 2693) oder sind Gegenstand eigener noch nicht veröffentlichter Patentanmeldungen (DE-P 3 221 215, DE-P 3 220 524, DE-P 3 220 523 und DE-P 3 356 598).

Man erhält sie beispielsweise, wenn man Ketone der Formel (IX),

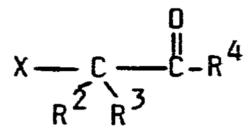

(IX)

in welcher

$R^2$, $R^3$, $R^4$ und X die oben angegebene Bedeutung haben,

mit Hydroxylamin-Derivaten der Formel (X),

$$R^5\text{-O-}NH_2 \qquad (X)$$

in welcher

$R^5$ die oben angegebene Bedeutung hat,

oder mit deren Hydrosalzen, gegebenenfalls in Gegenwart eines Verdünnungsmittels, wie beispielsweise Ethanol, und gegebenfalle in Gegenwart eines Säurebindemittels, wie beispielsweise Kaliumcarbonat, bei Temperaturen zwischen 0 °C und +120 °C umsetzt.

Chlorsulfonylisocyanat der Formel (VI), 2-Carboxyphenylsulfonsäureamide der Formel (VII), Ketone der Formel (IX) und Hydroxylamin-Derivate der Formel (X) ebenso wie deren Hydrosalze sind allgemein bekannte Verbindungen der organischen Chemie.

Als Verdünnungsmittel zur Durchführung der erfindungsgemäßen Verfahren (a) und (b) kommen vorzugsweise inerte organische Lösungsmittel infrage. Hierzu gehören insbesondere aliphatische oder aromatische, gegebenenfalls chlorierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan oder Tetrahydrofuran, Ketone, wie Aceton oder Butanon, Nitrile, wie Acetonitril oder Propionitril, Amide, wie Dimethylformamid, Ester, wie Essigsäureethylester oder Sulfoxide, wie Dimethylsulfoxid.

Die erfindungsgemäßen Verfahren (a) und (b) können gegebenenfalls in Gegenwart einer Base durchgeführt werden. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man zwischen -20 °C und +120 °C, vorzugsweise zwischen 0 °C und +50 °C.

Zur Durchführung des erfindungsgemäßen Verfahrens (a) setzt man pro Mol an heterocyclischer Aminoverbindung der Formel (II) im allgemeinen 1.0 bis 1.5 Mol, vorzugsweise 1.0 bis 1.2 Mol an 2-(Alkoximinoalkyloxycarbonyl)-phenylsulfonyliso(thio)cyanat der Formel (III), sowie gegebenenfalls 0.01 bis 1.0 Mol, vorzugsweise 0.1 bis 1.0 Mol an Base ein.

Zur Durchführung des erfindungsgemäßen Verfahrens (b) setzt man pro Mol an 2-(Alkoximinoalkyloxycarbonyl)-phenylsulfonamid der Formel (IV) im allgemeinen 1.0 bis 1.5 Mol, vorzugsweise 1.0 bis 1.2 Mol an (Thio)Carbamat der Formel (V), sowie gegebenenfalls 0.05 bis 1.5 Mol an, vorzugsweise 0.1 bis 1.0 Mol Base ein.

Die Aufarbeitung und Isolierung der Endprodukte der Formel (I) erfolgt bei allen Herstellungsverfahren und Varianten in üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer guten allgemein-herbiziden Wirkung gegenüber wichtigen Schadpflanzen auch eine gute Verträglichkeit gegenüber wichtigen Kulturpflanzen und können daher als Unkrautbekämpfungsmittel z. B. in Baumwolle oder Getreide eingesetzt werden.

Daneben besitzen die erfindungsgemäßen Wirkstoffe in entsprechenden Aufwandmengen auch eine starke mikrobizide Wirkung und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten wie beispielsweise gegen den Erreger der Reisfleckenkrankheit (Pyricularia oryzae) einsetzen. Dabei zeigen die erfindungsgemäßen Stoffe neben einer sehr guten protektiven Wirksamkeit auch hervorragende systemische Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Verwendung als Herbizide können die erfingungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; und 4-Amino-6-(1,1-dimethyl-ethyl)-3-methylthio-1,2,4-triazin-5(4H)-on zur Unkrautbekämpfung in Sojabohnen, in Frage. Auch Mischungen mit N,N-Dimethyl-N'-(3-trifluormethylphenyl)-harnstoff, N,N-Dimethyl-N'-(3-chlor-4-methylphenyl)-harnstoff, N,N-Dimethyl-N'-(isopropylphenyl)-harnstoff, 4-Amino-6-t-butyl-3-ethylthio-1,2,4-triazin-5(4H)-on, Chloressigsäure-N-(methoxymethyl)-2,6-diethylanilid, 2-Ethyl-6-methyl-N-(1-methyl-2-methoxyethyl)-chloracetanilid, 2,6-Dinitro-4-trifluormethyl-N,N-dipropylanilin, 2-[4-(3,5-Dichlorpyrid-2-yloxy)-phenoxy]-propionsäure-(2-benzyloxyethylester), -(trimethylsilylmethylester) oder -(2,2-diethoxyethylester) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 5 kg pro ha.

Bei der Verwendung als Fungizide können die erfindungsgemäßen Wirkstoffe in den Formulierungen oder in den verschiedenen Anwendungsformen ebenfalls in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die nachfolgenden Beispiele dienen zur weiteren Erläuterung der Erfindung, ohne diese einzuschränken.

## Herstellungsbeispiele:

### Beispiel 1:

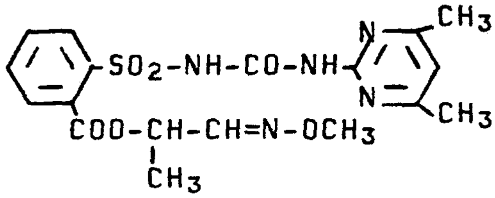

Zu einer Mischung von 58 g (0.2 Mol) 2-[1-(Methoximino)prop-2-yl-oxycarbonyl]-phenylsulfonsäureamid, 50 g (0.2 Mol) O-Phenyl-N-(4,6-dimethylpyrimidin-2-yl)-carbamat und 300 ml Acetonitril gibt man unter Rühren 31 g (0.2 Mol) Diazabicycloundecen (DBU) und rührt nach beendeter Zugabe weitere 20 Stunden bei Raumtemperatur. Zur Aufarbeitung gießt man den Reaktionsansatz in 1400 ml Wasser, klärt mit Aktivkohle, und säuert dann mit 20 ml konzentrierter Salzsäure an. Nach 2 Stunden bei 0 °C wird der Niederschlag abgesaugt und auf Ton getrocknet. Man erhält 54 g (62 % der Theorie) an N-(4,6-Dimethylpyrimidin-2-yl)-N'-[2-(1-Methoximinoprop-2-yl-oxycarbonyl)-phenylsulfonyl]-harnstoff vom Schmelzpunkt 118 °C - 127 °C.

### Herstellung der Ausgangsverbindung:

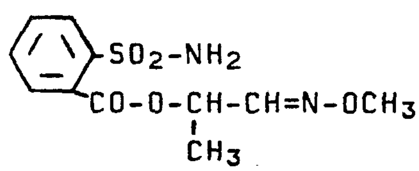

Zu einer Suspension von 100 g (0.5 Mol) 2-Sulfonamidobenzoesäure in 500 ml Acetonitril tropft man unter Rühren 51 g (0.5 Mol) Triethylamin und erhitzt anschließend für 60 Minuten auf 70 °C, danach für weitere 15 Minuten auf 75 °C - 80 °C, fügt dann 85 g (0.51 Mol) 2-Brom-1-methoximino-propan

zu und rührt weitere 20 Stunden bei 80 °C. Zur Aufarbeitung entfernt man das Lösungsmittel im Vakuum, nimmt den Rückstand in Methylenchlorid auf, wäscht zweimal mit Wasser, trocknet über Natriumsulfat und entfernt das Lösungsmittel im Vakuum. Man erhält 98 g (68,5 % der Theorie) an 2-[1-(Methoximinoprop-2-yl-oxycarbonyl]-phenylsulfonsäureamid vom Schmelzpunkt 115 °C - 120 °C.

Beispiel 2:

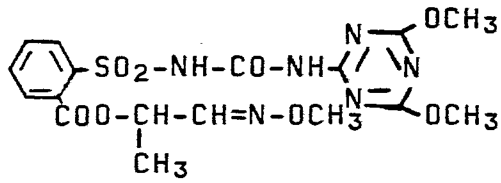

4,8 g (0.03 Mol) 2-Amino-4,6-dimethoxy-1,3,5-triazin, 10 g (0.03 Mol) 2-(1-Methoximinoprop-2-yloxycarbonyl)-phenylsufonylisocyanat und 4,5 g (0.03 Mol) Diazabicycloundecen (DBU) in 100 ml Acetonitril werden 20 Stunden gerührt, zur Aufarbeitung in 500 ml Wasser gegossen, mit konzentrierter Salzsäure angesäuert und mehrfach mit Essigester extrahiert. Die vereinigten organischen Phasen werden über Natriumsulfat getrocknet und im Vakuum vom Lösungsmittel befreit. Man erhält 2,2 g ( 24 % der Theorie) an N-(4,6-Dimethoxy-1,3,5-triazin-2-yl)-N'-[2-(1-Methoximinoprop-2-yloxycarbonyl)-phenylsulfonyl]-harnstoff vom Schmelzpunkt 160 °C - 168 °C.

Herstellung der Ausgangsverbindung:

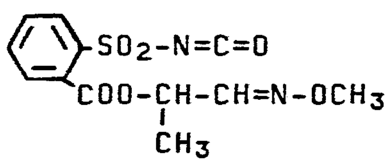

Zu einer Mischung von 29 g (0.1 Mol) 2-(1-Methoximinoprop-2-yloxycarbonyl)-phenylsulfonsäureamid und 1 g (0.01 Mol) Diazabicyclooctan (DABCO) in 300 ml Toluol tropft man unter Rühren 15 g (0.1 Mol) Chlorsulfonylisocyanat und erwärmt nach beendeter Zugabe innerhalb von einer Stunde auf 90 °C bis 95 °C, rührt weitere 2 Stunden bei dieser Temperatur, läßt abkühlen, dekantiert vom ungelösten Rückstand und engt im Vakuum ein. Man erhält 18 g (58 % der Theorie) an 2-(1-Methoximinoprop-2-yloxycarbonyl)-phenylsulfonylisocyanat als Oel.

IR: $\nu = 2200\ cm^{-1}$ (-N=C=O)

In entsprechender Weise und gemäß den allgemeinen Herstellungsangaben erhält man die folgenden 2-(Alkoximinoalkyloxycarbonyl)-phenylsulfonylharnstoffe der allgemeinen Formel (I):

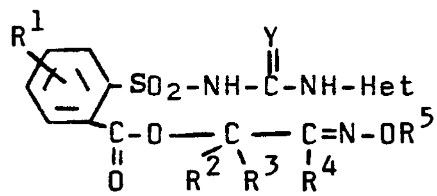

(I)

**Tabelle 2:**

| Bsp.-Nr. | $R^1$ | $R^2$ | $R^3$ | $R^4$ | $R^5$ | Y | Het | physikalische Eigenschaften |
|---|---|---|---|---|---|---|---|---|
| 3 | H | H | H | H | $CH_3$ | O | 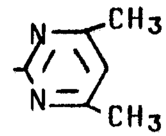 | Fp. 120° - 122 °C |
| 4 | H | H | H | $CH_3$ | $CH_3$ | O | 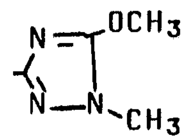 | Fp. 165° - 167 °C |
| 5 | H | H | H | $Ch_3$ | $CH_3$ | O | 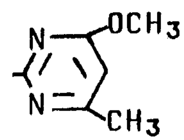 | Fp. 135° - 141 °C |
| 6 | H | H | H | $CH_3$ | $CH_3$ | O | 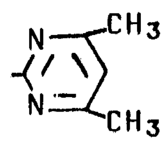 | Fp. 148° - 150 °C |
| 7 | H | $CH_3$ | H | H | $CH_3$ | O | 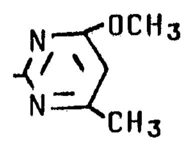 | Fp. 158° - 160 °C |
| 8 | H | $CH_3$ | H | H | $CH_3$ | O | 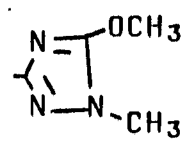 | Fp. 183° - 185 °C |

Anwendungsbeispiele:

In den folgenden Anwendungsbeispielen werden die nachstehend aufgeführten Verbindungen als Vergleichssubstanzen eingesetzt:

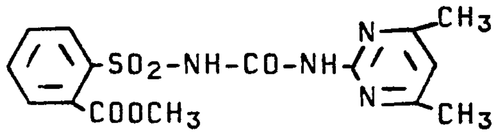

(A)

N-[2-(Methoxycarbonyl)-phenylsulfonyl]-N'-(4,6-dimethylpyrimidin-2-yl)-harnstoff

(bekannt aus EP-OS 34 431 oder US-PS 4 394 506)

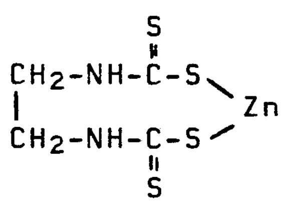

(B)

Zink-ethylen-1,2-bis(dithiocarbamat)

(bekannt aus R. Wegler, 'Chemie der Pflanzenschutz- und Schädlingsbekämpfungsmittel', Springer Verlag Berlin, Heidelberg, New York 1970, Band 2, S. 56 f)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Kulturpflanzenselektivität bei vergleichbarer herbizider Wirkung zeigt in diesem Test beispielsweise die Verbindung gemäß Herstellungsbeispiel 1.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß folgendem Herstellungsbeispiel: (1).

Beispiel C

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: (1)

0159576

Patentansprüche

1. 2-(Alkoximinoalkyloxycarbonyl)-phenylsulfonylharnstoffe der allgemeinen Formel (I)

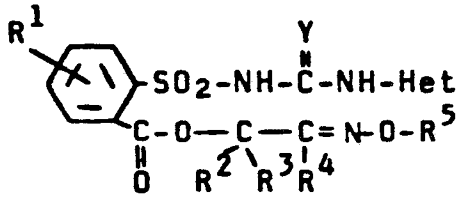

(I)

in welcher

$R^1$ für Wasserstoff, Halogen, Alkyl, Halogenalkyl, Alkoxy oder Nitro steht,

$R^2$ für Wassrstoff, Alkyl oder gegebenenfalls substituiertes Aryl steht,

$R^3$ für Wasserstoff oder Alkyl steht,

$R^4$ für Wasserstoff, Alkyl, Alkoxy, Alkylthio, Alkoxyalkyl, Alkylthioalkyl, Cyano, Alkoxycarbonyl oder gegebenenfalls substituiertes Aryl steht,

$R^5$ für Wasserstoff, Alkyl, Alkenyl, Alkinyl, Halogenalkyl, Halogenalkenyl, Alkoxyalkyl, Alkylthioalkyl oder für gegebenenfalls substituiertes Aralkyl steht,

Y für Sauerstoff oder Schwefel steht und

Het für einen heterocyclischen Rest der Formel

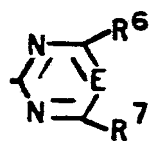

oder

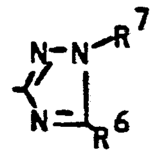

steht, wobei

E für Stickstoff oder die CH-Gruppe steht und

$R^6$ und $R^7$ unabhängig voneinander jeweils für Alkyl, Alkoxy oder Alkoxyalkyl stehen.

2. 2-(Alkoximinoalkyloxycarbonyl)-phenylsulfonylharnstoffe der allgemeinen Formel (I) gemäß Anspruch 1, dadurch gekennzeichnet, daß darin

$R^1$ für Wasserstoff, Fluor, Chlor, Brom, Jod, Trifluormethyl, Nitro, sowie jeweils geradkettiges oder verzweigtes Alkyl oder Alkoxy mit jeweils bis zu 4 Kohlenstoffatomen steht,

$R^2$ für Wasserstoff, für geradkettiges oder verzweigtes Alkyl mit bis zu 8 Kohlenstoffatomen oder für einfach oder mehrfach, gleich oder verschieden substituiertes Aryl mit 6 bis 10 Kohlenstoffatomen steht, wobei als Arylsubstituenten in Frage kommen:

Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen,

$R^3$ für Wasserstoff oder für geradkettiges oder verzweigtes Alkyl mit bis zu 4 Kohlenstoffatomen steht,

$R^4$ für Wasserstoff, Cyano oder für jeweils geradkettiges oder verzweigtes Alkyl, Alkoxy, Alkylthio, Alkoxyalkyl, Alkylthioalkyl oder Alkoxycarbonyl mit jeweils bis zu 6 Kohlenstoffatomen in den einzelnen Alkylteilen oder für gegebenenfalls einfach oder mehrfach gleich oder verschieden substituiertes Aryl mit 6 bis 10 Kohlenstoffatomen steht, wobei als Arylsubstituenten die bei $R^2$ genannten in Frage kommen,

$R^5$ für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 6 Kohlenstoffatomen in den einzelnen Alkyl- bzw. Alkenyl- oder Alkinylteilen und gegebenenfalls bis zu 9 gleichen oder verschiedenen Halogenatomen steht oder für geradkettiges oder verzweigtes, gegebenenfalls im Arylteil einfach oder mehrfach, gleich oder verschieden substituiertes

0159576

Aralkyl mit bis zu 4 Kohlenstoffatomen im Alkylteil und 6 bis 10 Kohlenstoffatomen im Arylteil steht, wobei als Arylsubstituenten die bei $R^2$ genannten in Frage kommen,

Y für Sauerstoff oder Schwefel steht und

Het für einen heterocyclischen Rest der Formel

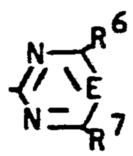

oder

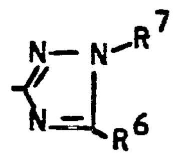

steht, wobei

E für Stickstoff oder die CH-Gruppe steht und

$R^6$ und $R^7$ unabhängig voneinander für jeweils geradkettiges oder verzweigtes Alkyl, Alkoxy oder Alkoxyalkyl mit jeweils bis zu 6 Kohlenstoffatomen in den einzelnen Alkylteilen stehen.

3. 2-(Alkoximinoalkyloxycarbonyl)-phenylsulfonylharnstoffe der allgemeinen Formel (I) gemäß Anspruch 1, dadurch gekennzeichnet, daß darin

$R^1$ für Wasserstoff, Fluor, Chlor, Brom, Nitro, Methyl, Ethyl, n- oder i-Propyl, Trifluormethyl, Methoxy oder Ethoxy steht,

$R^2$ für Wasserstoff, Methyl, Ethyl, n- oder i-Propyl, n-, i-, s- oder t-Butyl, sowie für gegebenenfalls ein- bis dreifach gleich oder verschieden durch Fluor, Chlor, Brom, Cyano, Methyl, Ethyl, Methoxy, Ethoxy oder Trifluormethyl substituiertes Phenyl steht,

$R^3$ für Wasserstoff, Methyl, Ethyl, n- oder i-Propyl, n-, i-, s- oder t-Butyl steht,

$R^4$ für Wasserstoff, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methoxymethyl, Methoxyethyl, Ethoxyethyl, Methylthiomethyl, Methylthioethyl, Ethylthioethyl, Methoxycarbonyl, Ethoxycarbonyl sowie für gegebenenfalls ein- bis dreifach gleich oder verschieden durch Fluor, Chlor, Brom, Cyano, Nitro, Methyl, Ethyl, Methoxy, Ethoxy oder Trifluormethyl substituiertes Phenyl steht,

$R^5$ für Wasserstoff, Methyl, Ethyl, n- oder i-Propyl, n-, i-, s- oder t-Butyl, Allyl, Butenyl, Propargyl, Methoxymethyl, Methoxyethyl, Ethoxyethyl, Methylthiomethyl, Methylthioethyl, Ethylthioethyl, Chlorallyl, Dichlorallyl oder für jeweils gegebenenfalls ein- bis dreifach, gleich oder verschieden im Phenylteil durch

Fluor, Chlor, Brom, Cyano, Nitro, Methyl, Ethyl, Methoxy, Ethoxy oder Trifluormethyl substituiertes Benzyl oder Phenylethyl steht,

Y für Sauerstoff oder Schwefel steht und

Het für einen heterocyclischen Rest der Formel

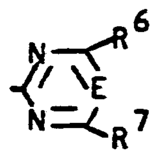

oder

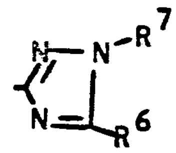

steht, wobei

E für Stickstoff oder die CH-Gruppe steht und

$R^6$ und $R^7$ unabhängig voneinander jeweils für Methyl, Ethyl, Methoxy, Ethoxy, Methoxymethyl, Ethoxymethyl, Methoxyethyl oder Ethoxyethyl stehen.

4. Verfahren zur Herstellung von 2-(Alkoximinoalkyloxycarbonyl)-phenylsulfonylharnstoffen der allgemeinen Formel (I) gemäß Anspruch 1, dadurch gekennzeichnet, daß man

(a) heterocyclische Aminoverbindungen der Formel (II),

$$\text{Het-NH}_2 \qquad \text{(II)}$$

in welcher

Het die in Anspruch 1 angegebene Bedeutung hat,

mit 2-(Alkoximinoalkyloxycarbonyl)-phenylsulfonyl-iso-(thio)cyanaten der Formel (III),

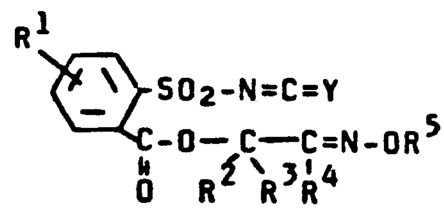

(III)

in welcher

$R^1$, $R^2$, $R^3$, $R^4$, $R^5$ und Y die in Anspruch 1 angegebene Bedeutung haben,

gegebenenfalls in Gegenwart eines Verdünnungsmittels und gegebenenfalls in Gegenwart eines basischen Katalysators umsetzt, oder daß man

(b) 2-(Alkoximinoalkyloxycarbonyl)-phenylsulfonamide der Formel (IV),

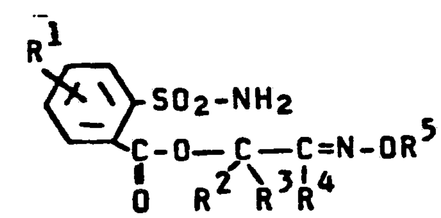

(IV)

in welcher

$R^1$, $R^2$, $R^3$, $R^4$ und $R^5$ die in Anspruch 1 angegebene Bedeutung haben,

mit (Thio)Carbamaten der Formel (V),

$$R^8-O-\overset{Y}{\overset{\|}{C}}-NH-Het \qquad (V)$$

in welcher

Y und Het die in Anspruch 1 angegebene Bedeutung haben und

$R^8$ für Alkyl oder Aryl steht,

jeweils gegebenenfalls in Gegenwart eines Verdünnungsmittels und jeweils gegebenenfalls in Gegenwart einer Base umsetzt.

5. Herbizide Mittel, gekennzeichnet durch einen Gehalt an mindestens einem 2-(Alkoximinoalkyloxycarbonyl)-phenyluslfonylharnstoff der Formel (I) gemäß Anspruch 1.

6. Verwendung von 2-(Alkoximinoalkyloxycarbonyl)-phenylsulfonylharnstoffen der allgemeinen Formel (I) gemäß Anspruch 1 zur Bekämpfung von unerwünschtem Pflanzenwachstum.

7. Fungizide Mittel, gekennzeichnet durch einen Gehalt an mindestens einem 2-(Alkoximinoalkyloxycarbonyl)-phenylsulfonylharnstoff der Formel (I) gemäß Anspruch 1.

8. Verwendung von 2-(Alkoximinoalkyloxycarbonyl)-phenylsulfonylharnstoffen der Formel (I) gemäß Anspruch 1 zur Bekämpfung von unerwünschten Mikroorganismen.

9. Verfahren zur Herstellung von herbiziden bzw. fungiziden Mitteln, dadurch gekennzeichnet, daß man 2-(Alkoximinoalkyloxycarbonyl)-phenylsulfonylharnstoffe der allgemeinen Formel (I) gemäß Anspruch 1 mit Streckmitteln und/oder oberflächenaktiven Mitteln vermischt.